# EUROPÄISCHE PATENTSCHRIFT

(11) **EP 4 511 178 B1**
(45) Veröffentlichungstag und Bekanntmachung des Hinweises auf die Patenterteilung: **17.09.2025**
(21) Anmeldenummer: 24723051.9
(22) Anmeldetag: 26.04.2024
(51) Int. Cl.: B05C 5/02, B05C 11/10, B05C 9/14, B05C 13/02, A61L 29/04, B05C 1/02

(54) **VERFAHREN UND VORRICHTUNG ZUM BESCHICHTEN EINER MEDIZINISCHEN INVASIVKOMPONENTE**
METHOD AND DEVICE FOR COATING A MEDICAL INVASIVE COMPONENT
PROCÉDÉ ET DISPOSITIF DESTINÉS AU REVÊTEMENT D'UN ÉLÉMENT INVASIF MÉDICAL

(30) Priorität: 28.04.2023 DE 102023111000
(43) Veröffentlichungstag der Anmeldung: 26.02.2025
(73) Patentinhaber: B. Braun Melsungen AG, 34212 Melsungen (DE)
(72) Erfinder: ENGELIEN, Eberhard, 21521 Wohltorf (DE)
(74) Vertreter: Patentanwälte Ruff, Wilhelm, Beier, Dauster & Partner mbB
(86) Internationale Anmeldenummer: PCT/EP2024/061599
(87) Internationale Veröffentlichungsnummer: WO 2024/223862

(56) Entgegenhaltungen:
- EP-A1- 3 106 197
- US-A1- 2004 247 775
- US-B2- 8 888 498

## Beschreibung

Die Erfindung betrifft eine Vorrichtung mit den oberbegrifflichen Merkmalen des Anspruchs 1 und ein Verfahren mit den oberbegrifflichen Merkmalen des Anspruchs 8.

Medizinische Invasivkomponenten werden zur Verbesserung ihrer funktionellen Eigenschaften oftmals mit einer Beschichtung versehen.

Beispielsweise werden Katheterschläuche in der Regel mit einer hydrophilen Beschichtung versehen, die bei Kontakt mit Flüssigkeit einen Gleitfilm bildet, der die Gleiteigenschaften des Katheterschlauchs in einem Führungsschlauch oder im Körperinneren verbessern soll.

Aus dem Stand der Technik sind unterschiedliche Verfahren zur hydrophilen Beschichtung von Katheterschläuchen bekannt.

Bei einem bekannten Verfahren wird eine viskose Beschichtungslösung auf die Mantelfläche des Katheterschlauchs aufgesprüht. Die viskose Beschichtungslösung umfasst eine flüchtige Lösungskomponente, nach deren Verdunsten der eigentliche Beschichtungsstoff in Form der hydrophilen Beschichtung auf der Mantelfläche verbleibt. Bereiche der Mantelfläche, die nicht beschichtet werden sollen, müssen vor dem Aufsprühen abgedeckt werden. Zudem muss bei solchen Aufsprühverfahren mehr viskose Beschichtungslösung aufgewendet werden, als letztlich auf die Mantelfläche gelangt.

Bei einem weiteren bekannten Verfahren wird der Katheterschlauch in die viskose Beschichtungslösung eingetaucht, was wiederum ein Abdecken nicht zu beschichtender Bereiche voraussetzt. Auch bei solchen Tauchverfahren muss eine deutlich größere Menge der viskosen Beschichtungslösung bereitgestellt werden, als letztlich auf die Mantelfläche gelangt.

Bei einem weiteren bekannten Verfahren wird die viskose Beschichtungslösung mit einem Werkzeug, beispielsweise einem Schwamm oder Pinsel, auf die Mantelfläche aufgestrichen. Die aufstreichbare Menge an Beschichtungslösung ist durch die Anpresskraft des Werkzeugs begrenzt. Die Schichtdicke der Beschichtung ist nicht oder allenfalls schlecht einstellbar.

Die EP 3 106 197 A1 offenbart eine Vorrichtung und ein Verfahren mit den oberbegrifflichen Merkmalen der Ansprüche 1 und 8.

Die US 2004/247775 A1 offenbart eine Vorrichtung und ein Verfahren zum Beschichten mit einem Verdrängungsbeschichtungsgerät, bei welchem eine Durchflussmenge der Beschichtungslösung gesteuert wird, wobei sich etwaige Unterschiede in der Viskosität der Beschichtungslösung nicht nachteilig auf die abgegebene Menge der Beschichtungslösung auswirken sollen.

Aufgabe der Erfindung ist es, ein Verfahren und eine Vorrichtung bereitzustellen, die eine verbesserte Beschichtung von medizinischen Invasivkomponenten mit einer rotationssymmetrischen sowie gerade längserstreckten Mantelfläche erlaubt. Insbesondere soll eine möglichst vollflächige Beschichtung ausgewählter Bereiche mit gut einstellbarer Schichtdicke bei minimiertem Verbrauch von viskoser Beschichtungslösung erreicht werden.

Diese Aufgabe wird durch das Bereitstellen einer Vorrichtung mit den Merkmalen des Anspruchs 1 und eines Verfahrens mit den Merkmalen des Anspruchs 8 gelöst. Vorteilhafte Ausgestaltungen sind in den Unteransprüchen angegeben.

Die erfindungsgemäße Vorrichtung weist eine Rotationseinrichtung, eine Aufbringeinrichtung, eine Linearbewegungseinrichtung und eine Steuereinrichtung auf. Die Rotationseinrichtung ist zum Einspannen und angetriebenen Rotieren der Invasivkomponente um deren Längsachse eingerichtet. Die Rotationseinrichtung ist zum Rotieren der Invasivkomponente mit einer definierten Drehzahl eingerichtet. Die Drehzahl ist ein Maß für eine Anzahl der Umdrehungen der Rotationseinrichtung und damit auch der Invasivkomponente pro Zeiteinheit. Die Aufbringeinrichtung ist relativ zu der Längsachse rotatorisch unbeweglich und zum Aufbringen einer viskosen Beschichtungslösung auf die Mantelfläche der rotierenden Invasivkomponente eingerichtet. Die Aufbringeinrichtung ist zum Aufbringen der viskosen Beschichtungslösung mit einer definierten Aufbringrate eingerichtet. Die Aufbringrate ist ein Maß für ein pro Zeiteinheit aufgebrachtes Volumen oder Gewicht der viskosen Beschichtungslösung. Bei einer Ausgestaltung ist die Aufbringeinrichtung in Bezug auf die Gravitationsrichtung unterhalb der Längsachse angeordnet ("stehender Tropfen der viskosen Beschichtungslösung"). Bei einer weiteren Ausgestaltung ist die Aufbringeinrichtung stattdessen oberhalb der Längsachse angeordnet ("hängender Tropfen der viskosen Beschichtungslösung"). Die Linearbewegungseinrichtung ist zur angetriebenen Linearbewegung der Aufbringeinrichtung und/oder der Rotationseinrichtung entlang der Längsachse der rotierenden Invasivkomponente eingerichtet. Mittels der Linearbewegungseinrichtung sind die Aufbringeinrichtung und die Rotationseinrichtung relativ zueinander angetrieben linearbeweglich. Bei einer Ausgestaltung ist die Linearbewegungseinrichtung zur Linearbewegung der Aufbringeinrichtung eingerichtet, wobei die Rotationseinrichtung linear unbeweglich ist. Bei einer weiteren Ausgestaltung ist die Linearbewegungseinrichtung zur Linearbewegung der Rotationseinrichtung eingerichtet, wobei die Aufbringeinrichtung linear unbeweglich ist. Bei einer weiteren Ausgestaltung ist die Linearbewegungseinrichtung zur Linearbewegung sowohl der Aufbring- als auch der Rotationseinrichtung eingerichtet. Die Linearbewegungseinrichtung ist zur Linearbewegung mit einer definierten Vorschubgeschwindigkeit eingerichtet. Die Vorschubgeschwindigkeit ist ein Maß für eine von der Aufbringeinrichtung und/oder der Rotationseinrichtung pro Zeiteinheit entlang der Längsachse zurückgelegte Strecke. Die Steuereinrichtung ist zum Steuern der Drehzahl der Rotationseinrichtung, der Aufbringrate der Aufbringeinrichtung und der Vorschubgeschwindigkeit der Linearbewegungseinrichtung eingerichtet. Die Steuerung erfolgt automatisch. Die Steuereinrichtung kann zu diesem Zweck drahtlos oder drahtgebunden mit der Rotations-, Aufbring- und Linearbewegungseinrichtung verbunden sein. Zum Aufbringen der Beschichtung wird die Invasivkomponente zunächst in die Rotationseinrichtung eingespannt. Die Rotationseinrichtung ist bei unterschiedlichen Ausgestaltungen auf unterschiedliche Weise zum Einspannen der Invasivkomponente eingerichtet. Beispielsweise kann die Rotationseinrichtung ein Spanneinheit mit beweglichen Spannbacken oder dergleichen aufweisen. Hiernach wird die eingespannte Invasivkomponente um ihre Längsachse angetrieben rotiert. Zu diesem Zweck weist die Rotationseinrichtung vorzugsweise eine Antriebseinheit auf, insbesondere einen Elektroantrieb. Die besagte Antriebseinheit ist jedoch nicht bei sämtlichen Ausgestaltungen Bestandteil der Vorrichtung. Weiter wird die Aufbringeinrichtung und/oder die Rotationseinrichtung mittels der Linearbewegungseinrichtung unter gleichzeitigem Aufbringen der Beschichtungslösung entlang der Längsachse der rotierenden Invasivkomponente bewegt. Zum Aufbringen der viskosen Beschichtungslösung mit der definierten Aufbringrate weist die Aufbringeinrichtung bei einer Ausgestaltung wenigstens eine Aufbringeinheit und eine Pumpeinheit auf. Die optionale Aufbringeinheit kann beispielsweise in Form einer einends mit einer Austrittsöffnung versehenen Hohlnadel, eines Röhrchens, eines verjüngten Schlauchs oder dergleichen gestaltet sein und dient dem Applizieren der viskosen Beschichtungslösung auf die Mantelfläche. Die Aufbringeinrichtung, im Speziellen die Aufbringeinheit, ist vorzugsweise derart radial von der Längsachse beabstandet, dass die rotierende Mantelfläche die viskose Beschichtungslösung gleichsam von der Aufbringeinrichtung/Aufbringeinheit abzieht. Der besagte Abstand beträgt vorzugsweise zwischen 0,1 mm und 1,5 mm, besonders bevorzugt zwischen 0,2 mm und 1,0 mm. Sofern die zu beschichtende Mantelfläche einen über der Längsachse veränderlichen Außendurchmesser aufweisen sollte, wird der Abstand vorzugsweise entsprechend konstant gehalten, beispielsweise mittels einer radialen Linearbewegungseinrichtung, die zur relativen radialen Verlagerung zwischen der Aufbring- und der Rotationseinrichtung eingerichtet ist. Die optionale Pumpeinheit dient einem Fördern der viskosen Beschichtungslösung durch die Aufbringeinheit, wobei die Aufbringrate über eine Veränderung einer Pumpleistung der Pumpeinheit beeinflusst werden kann. Die Pumpleistung und damit die Aufbringrate wird vorzugsweise derart gesteuert, dass die Aufbringeinrichtung, im Speziellen die Aufbringeinheit, einen (stehenden oder hängenden) Tropfen hervorbringt, der bei Kontakt mit der rotierenden Mantelfläche abgezogen wird, wodurch die Invasivkomponente von der viskose Beschichtungslösung umwickelt wird. Bei einer weiteren Ausgestaltung ist die besagte Pumpeinheit abseits der Aufbringeinrichtung angeordnet und folglich relativ zu dieser feststehend. Beispielsweise kann die Pumpeinheit im Bereich eines Lösungsbehälters angeordnet sein, der die aufzubringende viskose Beschichtungslösung bevorratet. Die Bewegung erfolgt mittels der Linearbewegungseinrichtung. Die viskose Beschichtungslösung wird hierbei spiralförmig, d.h. wendel- und/oder helixförmig, auf die sich relativ zu der Aufbringeinrichtung rotatorisch bewegende Mantelfläche aufgebracht oder auch aufgewickelt. Die Linearbewegung der Aufbringeinrichtung kann über eine gesamte Länge der Mantelfläche oder lediglich über einen Teil, d.h. ausgewählte Bereiche, derselben erfolgen. Die auf diese Weise auf die Mantelfläche aufgebrachte viskose Beschichtungslösung bildet - nach einem Abtrocknen, Verdunsten und/oder Vernetzen - die eigentliche Beschichtung aus. Ob zum Ausbilden der Beschichtung ein Abtrocknen, Verdunsten und/oder Vernetzen der viskosen Beschichtungslösung erfolgt, hängt ab von den Eigenschaften der verwendeten Beschichtungslösung/Beschichtung und ist nicht wesentlich für den Kern der Erfindung. Die Homogenität, Vollflächigkeit und/oder Schichtdicke der Beschichtung kann auf einfache Weise durch eine aufeinander abgestimmte Steuerung der Drehzahl, der Aufbringrate und der Vorschubgeschwindigkeit eingestellt werden. Zudem ist es denkbar und möglich, dass ein mehrfacher Vorschub in entgegengesetzte Vorschubrichtungen erfolgt, so dass die Beschichtung gleichsam durch mehrere Lagen der viskosen Beschichtungslösung gebildet wird. Gemäß der Erfindung ist die Steuereinrichtung zum Steuern der Drehzahl, der Aufbringrate und der Vorschubgeschwindigkeit in Abhängigkeit der Viskosität der viskosen Beschichtungslösung eingerichtet. Die Viskosität der viskosen Beschichtungslösung bildet einen Steuerparameter der automatischen Steuerung. Bei einer Ausgestaltung der Erfindung weist die Vorrichtung eine Eingabeeinrichtung auf, die zur manuellen Eingabe einer die Viskosität repräsentierenden Eingabegröße eingerichtet ist. Bei einer weiteren Ausgestaltung der Erfindung weist die Vorrichtung eine Speichereinrichtung auf, in der Daten abgespeichert sind, die repräsentativ für unterschiedliche Viskositäten unterschiedlicher Beschichtungslösungen sind. Die Daten können in Abhängigkeit von der tatsächlich verwendeten viskosen Beschichtungslösung aus der Speichereinrichtung abgerufen und der Steuerung zugrunde gelegt werden. Denkbar und möglich ist es zudem, dass die Viskosität mittels einer Messeinrichtung im Betrieb der Vorrichtung ermittelt wird. Sofern die Mantelfläche einen über der Längsachse veränderlichen Außendurchmesser aufweist, ist die Steuereinrichtung vorzugsweise zum Steuern der Drehzahl, der Aufbringrate und der Vorschubgeschwindigkeit in Abhängigkeit der Viskosität und des veränderlichen Außendurchmesser eingerichtet. In diesem Fall können in der optionalen Speichereinrichtung Daten abgespeichert sein, die repräsentativ für den über der Längsachse veränderlichen Außendurchmesser sind. Weiter gemäß der Erfindung ist die Steuereinrichtung derart zu einem aufeinander abgestimmten Steuern der Drehzahl, der Aufbringrate und der Vorschubgeschwindigkeit eingerichtet, dass die viskose Beschichtungslösung vollflächig in Form einer entlang der Längsachse überlappenden Helix auf die Mantelfläche aufbringbar ist. Die Drehzahl, die Aufbringrate und die Vorschubgeschwindigkeit werden folglich derart aufeinander abgestimmt, dass die Mantelfläche in Umfangs- und Axialrichtung von der viskosen Beschichtungslösung durchgängig bedeckt wird. Bei einer Ausgestaltung erfolgt die insoweit abgestimmte Steuerung ausgehend von einer vorgegebenen Drehzahl, wobei die Aufbringrate und/oder die Vorschubgeschwindigkeit hieran angepasst ermittelt werden, beispielsweise auf Grundlage entsprechender Bestimmungsgleichungen, anhand von Kennfeldern oder dergleichen. Bei einer weiteren Ausgestaltung erfolgt die abgestimmte Steuerung ausgehend von einer vorgegebenen Aufbringrate, wobei die Drehzahl und die Vorschubgeschwindigkeit entsprechend ermittelt werden. Bei einer weiteren Ausgestaltung erfolgt die abgestimmte Steuerung ausgehend von einer vorgegebenen Vorschubgeschwindigkeit, wobei die Drehzahl und/oder die Aufbringrate entsprechend abgestimmt ermittelt werden. Es versteht sich, dass auch Kombinationen der vorgenannten Ansätze denkbar und möglich sind. Im Rahmen dieser Beschreibung umfassen durch Formulierungen wie "zwischen Wert X und Wert Y" definierte Wertebereiche ausdrücklich auch deren, hier nur beispielhaft genannte, Grenzwerte X und Y. Vorzugsweise weist die zu beschichtende Mantelfläche einen Außendurchmesser zwischen 0,5 mm und 1,5 mm, bevorzugt zwischen 0,7 mm und 1,0 mm, besonders bevorzugt zwischen 0,8 mm und 0,9 mm, auf. Bevorzugt ist der Außendurchmesser über der Längsachse konstant.

Die erfindungsgemäße Lösung eignet sich zur Beschichtung von medizinischen Invasivkomponenten mit einer Längsachse und einer um die Längsachse rotationssymmetrischen sowie gerade längserstreckten Mantelfläche. Besonders bevorzugt eignet sich die erfindungsgemäße Lösung zur hydrophilen Beschichtung von Katheterschläuchen.

In weiterer Ausgestaltung der Erfindung ist die aufzubringende viskose Beschichtungslösung aus mehreren separat bevorrateten Lösungskomponenten gebildet, wobei die Aufbringeinrichtung zum gleichzeitigen Aufbringen der mehreren Lösungskomponenten eingerichtet ist und für jede der mehreren Lösungskomponenten jeweils eine Aufbringeinheit aufweist. Diese Ausgestaltung der Erfindung erlaubt eine einfache und effiziente Applikation mehrkomponentiger Beschichtungslösungen. Die Aufbringeinheiten sind vorzugsweise jeweils in Form einer einends mit einer Austrittsöffnung versehenen Hohlnadel oder dergleichen gestaltet. Die unterschiedlichen Lösungskomponenten müssen nicht unbedingt gleichzeitig aufgebracht werden. Vielmehr ist es denkbar und möglich, dass zunächst eine der Lösungskomponenten entlang einer Vorschubrichtung und hiernach eine weitere Lösungskomponenten entlang einer entgegengesetzten Vorschubrichtung aufgebracht werden. Alternativ erfolgt das Aufbringen nacheinander in gleichgerichteter Vorschubrichtung, wodurch auf einfache Weise gewährleistet werden kann, dass beschichtete Bereiche der Mantelfläche vor dem Aufbringen einer weiteren Lösungskomponente gleiche Verdunstungs- und/oder Trocknungszeiten erhalten. Zwar ist es grundsätzlich möglich, dass auch mehrkomponentige Beschichtungslösungen mit lediglich einer Aufbringeinheit aufgebracht werden. Dies erfordert jedoch unter Umständen ein zwischenzeitliches Reinigen der Aufbringeinheit. Hierauf kann bei dieser Ausgestaltung der Erfindung verzichtet werden.

In weiterer Ausgestaltung der Erfindung ist eine Verdunstungseinrichtung vorhanden und zum Verdunsten und/oder Beschleunigen einer Verdunstung einer flüchtigen Lösungskomponente der auf die Mantelfläche aufgebrachten viskosen Beschichtungslösung eingerichtet. Insbesondere hydrophile Beschichtungslösungen weisen oftmals eine flüchtige Lösungskomponente auf, die als Lösungsmittel für einen eigentlichen Beschichtungsstoff dient. Nach dem Aufbringen der viskosen Beschichtungslösung ist ein Verdunsten der flüchtigen Lösungskomponente vorgesehen, so dass der Beschichtungsstoff unter Ausbildung der hydrophilen Beschichtung auf der Mantelfläche verbleibt. Die Verdunstungseinrichtung bewirkt und/oder unterstützt diesen Verdunstungsvorgang. Bei unterschiedlichen Ausgestaltungen ist die Verdunstungseinrichtung unterschiedlich gestaltet. Bei einer Ausgestaltung weist die Verdunstungseinrichtung eine Heizeinheit zum Aufheizen der mit der viskosen Beschichtungslösung versehenen Mantelfläche auf. Bei einer weiteren Ausgestaltung weist die Verdunstungseinrichtung alternativ oder zusätzlich eine Lüftereinheit auf, die zum Anblasen der mit der viskosen Beschichtungslösung versehenen Mantelfläche eingerichtet ist. Durch das Heizen und/oder Anblasen der auf der Mantelfläche befindlichen viskosen Beschichtungslösung kann der Verdunstungsprozess - je nach den spezifischen Eigenschaften der verwendeten viskosen Beschichtungslösung und der Umgebungsbedingungen - initiiert, aufrechterhalten und/oder beschleunigt werden.

In weiterer Ausgestaltung der Erfindung ist eine Vernetzungseinrichtung vorhanden und zum Vernetzen und/oder Beschleunigen einer Vernetzung von zu vernetzenden Lösungskomponenten der auf die Mantelfläche aufgebrachten viskosen Beschichtungslösung eingerichtet. Insbesondere hydrophile Beschichtungen werden oftmals unter Vernetzung von viskosen Lösungskomponenten ausgebildet. Dabei werden die verwendeten Lösungskomponenten vorzugsweise einzeln und nacheinander und nicht etwa miteinander vernetzt. Die Vernetzungseinrichtung ist zum Initiieren, Aufrechterhalten und/oder Beschleunigen des Vernetzungsprozesses eingerichtet und bei unterschiedlichen Ausgestaltungen der Erfindung unterschiedlich gestaltet. Beispielsweise sind mittels Lichts, insbesondere UV-Licht, vernetzbare Beschichtungslösungen bekannt. In diesem Fall weist die Vernetzungseinrichtung vorzugsweise wenigstens eine Lichtquelle auf, insbesondere eine UV-Lichtquelle.

In weiterer Ausgestaltung der Erfindung ist die Rotationseinrichtung eingerichtet zum Einspannen und angetriebenen Rotieren von Invasivkomponenten mit einem Durchmesser von 0,5 mm bis 10 mm, bevorzugt von 0,7 mm bis 0,9 mm, und einer Länge von 40 mm bis 500 mm, bevorzugt von 80 mm bis 250 mm, und mit einer Drehzahl von 20 U/min bis 900 U/min, bevorzugt von 250 U/min bis 800 U/min, bevorzugt mit einer Drehzahl von 300 U/min bis 600 U/min, bevorzugt von 600 U/min.

In weiterer Ausgestaltung der Erfindung ist die Aufbringeinrichtung eingerichtet zum Aufbringen viskoser Beschichtungslösungen mit einer Viskosität von 25 x 10⁻³ kg/ms bis 80 x 10⁻³ kg/ms, bevorzugt von 35 x 10⁻³ kg/ms bis 55 x 10⁻³ kg/ms, weiter bevorzugt von 43 x 10⁻³ kg/ms bis 48 x 10⁻³ kg/ms, mit einer Aufbringrate von 0,5 µl/s bis 10 µl/s, bevorzugt von 0,7 µl/s bis 5 µl/s, weiter bevorzugt von 1 µl/s bis 3 µl/s, weiter bevorzugt von 0,9 µl/s bis 2,5 µl/s. Die verarbeitbaren Viskositäten bewegen sich folglich zwischen 25 und 80 cP (Centipoise).

In weiterer Ausgestaltung der Erfindung ist die Linearbewegungseinrichtung eingerichtet zur angetriebenen Linearbewegung der Aufbringeinrichtung und/oder der Rotationseinrichtung mit einer Vorschubgeschwindigkeit zwischen 0,5 mm/s und 50 mm/s, bevorzugt zwischen 4 mm/s und 40 mm/s, weiter bevorzugt zwischen 5 mm/s und 10 mm/s, weiter bevorzugt 10 mm/s.

Das erfindungsgemäße Verfahren dient einem Beschichten einer medizinischen Invasivkomponente mit einer Längsachse und einer um die Längsachse rotationssymmetrischen sowie gerade längserstreckten Mantelfläche. Insbesondere dient das Verfahren einem hydrophilen Beschichten eines Katheterschlauchs. Das erfindungsgemäße Verfahren weist die Schritte auf: Einspannen der Invasivkomponente in eine Rotationseinrichtung; Rotieren der eingespannten Invasivkomponente mittels der Rotationseinrichtung, wobei die Rotation mit einer definierten Drehzahl erfolgt; Aufbringen einer viskosen Beschichtungslösung auf die Mantelfläche der rotierenden Invasivkomponente, wobei die viskose Beschichtungslösung mittels einer Aufbringeinrichtung, die relativ zu der Längsachse rotatorisch unbeweglich ist, und mit einer definierten Aufbringrate aufgebracht wird; lineares Bewegen der Aufbringeinrichtung und/oder der Rotationseinrichtung entlang der Längsachse der rotierende Invasivkomponente, wobei die Aufbringeinrichtung mittels einer Linearbewegungseinrichtung und mit einer definierten Vorschubgeschwindigkeit relativ zu der Rotationseinrichtung linear bewegt wird und/oder umgekehrt; Steuern der Drehzahl, der Aufbringrate und der Vorschubgeschwindigkeit mittels einer Steuereinrichtung, wobei die Drehzahl, die Aufbringrate und die Vorschubgeschwindigkeit in Abhängigkeit der Viskosität der viskosen Beschichtungslösung derart aufeinander abgestimmt gesteuert werden, dass die viskose Beschichtungslösung vollflächig in Form einer entlang der Längsachse überlappenden Helix auf die Mantelfläche aufgebracht wird. Das bereits zu der erfindungsgemäßen Vorrichtung Gesagte gilt, mutatis mutandis, auch für das erfindungsgemäße Verfahren. Zur Vermeidung von Wiederholungen wird folglich auf die Beschreibung der erfindungsgemäßen Vorrichtung ausdrücklich Bezug genommen und verwiesen. Ausgestaltungen des erfindungsgemäßen Verfahrens sind in den abhängigen Verfahrensansprüchen angegeben und ergeben sich zudem unmittelbar und eindeutig aus der Offenbarung der Ausgestaltungen der erfindungsgemäßen Vorrichtung. So können Ausgestaltungen des erfindungsgemäßen Verfahrens beispielsweise ein Aufbringen gemäß Anspruch 2, ein Verdunsten gemäß Anspruch 3 und/oder ein Vernetzen gemäß Anspruch 4 aufweisen.

In weiterer Ausgestaltung der Erfindung beträgt die Drehzahl zwischen 20 U/min und 900 U/min, bevorzugt zwischen 250 U/min und 800 U/min, bevorzugt zwischen 300 U/min bis 600 U/min, bevorzugt 600 U/min.

In weiterer Ausgestaltung der Erfindung beträgt die Viskosität zwischen 25 x 10⁻³ kg/ms und 80 x 10⁻³ kg/ms, bevorzugt zwischen 35 x 10⁻³ kg/ms und 55 x 10⁻³ kg/ms, weiter bevorzugt zwischen 43 x 10⁻³ kg/ms bis 48 x 10⁻³ kg/ms.

In weiterer Ausgestaltung der Erfindung beträgt die Aufbringrate zwischen 0,5 µl/s und 10 µl/s, bevorzugt zwischen 0,7 µl/s und 5 µl/s, weiter bevorzugt zwischen 1 µl/s und 3 µl/s, weiter bevorzugt zwischen 0,9 µl/s und 2,5 µl/s.

In weiterer Ausgestaltung der Erfindung beträgt die Vorschubgeschwindigkeit zwischen 0,5 mm/s und 50 mm/s, weiter bevorzugt zwischen 4 mm/s und 40 mm/s, bevorzugt zwischen 5 mm/s und 10 mm/s, weiter bevorzugt 10 mm/s.

In weiterer Ausgestaltung der Erfindung beträgt die Drehzahl zwischen 300 U/min und 600 U/min, die Viskosität beträgt zwischen 35 x 10⁻³ kg/ms und 55 x 10⁻³ kg/ms, die Aufbringrate beträgt zwischen 1 µl/s und 3 µl/s, und die Vorschubgeschwindigkeit beträgt zwischen 5 mm/s und 10 mm/s. Dies ist eine besonders bevorzugte Ausgestaltung. In weiterer Ausgestaltung der Erfindung beträgt die Drehzahl 600 U/min, die Viskosität beträgt zwischen 43 x 10⁻³ kg/ms und 48 x 10⁻³ kg/ms, die Aufbringrate beträgt zwischen 0,9 µl/s und 2,5 µl/s, und die Vorschubgeschwindigkeit beträgt 10 mm/s. Die genannten Wertebereiche der Verfahrensparameter haben sich als besonders vorteilhaft im Hinblick auf die Lösung der im Raum stehenden Aufgabe erwiesen. Diese Ausgestaltung der Erfindung gewährleistet insbesondere, dass - trotz der vergleichsweise hochviskosen und/oder pastösen Eigenschaften der Beschichtungslösung - keine Fehlstellen zwischen benachbarten Wendeln der Helix/Beschichtung verbleiben und sich eine entlang der Längsachse konstante und/oder homogene Schichtdicke der Beschichtung ergibt. Eine solch abgestimmte Steuerung der Verfahrensparameter wäre bei einer vergleichsweise niedrigviskosen Beschichtungsflüssigkeit nicht erforderlich, da diese benetzend ist und/oder auf der Mantelfläche verläuft und folglich in Form eines Flüssigkeitsfilms - und nicht etwa in Form einer Helix - aufbringbar ist.

In weiterer Ausgestaltung der Erfindung erfolgt zunächst eine Beschichtung mit einem Primer, der auch als Basiscoat bezeichnet werden kann, und hiernach eine Beschichtung mit einem Topcoat.

In weiterer Ausgestaltung der Erfindung wird die viskose Beschichtungslösung in Bezug auf die Gravitationsrichtung von oben nach unten in Form eines hängenden Tropfens an der Aufbringeinrichtung ausgebracht, wobei der hängende Tropfen von der rotierenden Invasivkomponente kontaktiert wird, wodurch deren Mantelfläche helixförmig von der viskosen Beschichtungslösung umwickelt wird. Die Aufbringrate wird zum Ausbringen des hängenden Tropfens abgestimmt gesteuert.

In weiterer Ausgestaltung der Erfindung wird die viskose Beschichtungslösung in Bezug auf die Gravitationsrichtung von unten nach oben in Form eines stehenden Tropfens an der Aufbringeinrichtung ausgebracht, wobei der stehende Tropfen von der rotierenden Invasivkomponente kontaktiert wird, wodurch deren Mantelfläche helixförmig von der viskosen Beschichtungslösung umwickelt wird. Die Aufbringrate wird zum Ausbringen des hängenden Tropfens abgestimmt gesteuert.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt in schematisch vereinfachter Darstellung eine Ausführungsform einer erfindungsgemäßen Vorrichtung zum Beschichten einer medizinischen Invasivkomponente in Form eines Katheterschlauchs,
- Fig. 2: in vergrößerter Detaildarstellung einen Abschnitt des Katheterschlauchs,
- Fig. 3: in schematisch vereinfachter Darstellung eine Variante einer Aufbringeinrichtung der Vorrichtung nach Fig. 1 und
- Fig. 4: ein Flussdiagramm zur Verdeutlichung einer Ausführungsform eines erfindungsgemäßen Verfahrens.

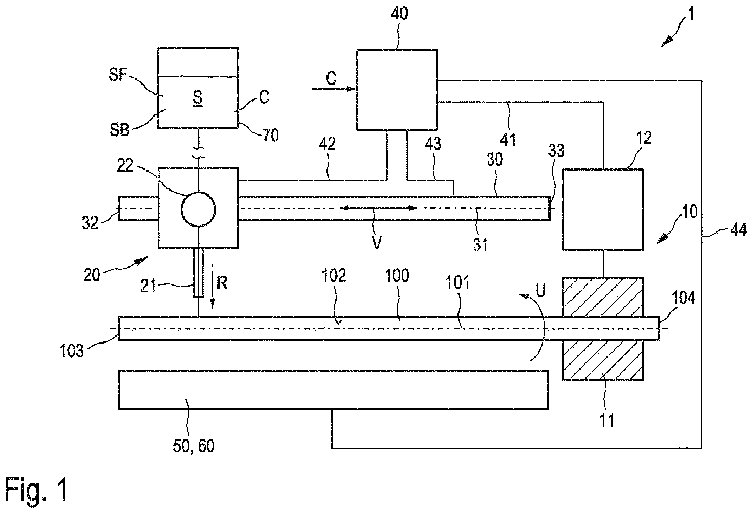

Gemäß Fig. 1 ist eine Vorrichtung 1 zum Beschichten einer medizinischen Invasivkomponente vorgesehen.

Bei der Invasivkomponente handelt es sich vorliegend um einen Katheterschlauch 100 mit einer Längsachse 101 und einer Mantelfläche 102. Die Mantelfläche 102 ist entlang der Längsachse 101 gerade längserstreckt und rotationssymmetrisch. Der zu beschichtende Katheterschlauch 100 ist zwischen einem ersten Ende 103 und einem zweiten Ende 104 längserstreckt, wobei die anhand Fig. 1 ersichtlichen Dimensionen als rein exemplarisch und nicht maßstäblich zu verstehen sind.

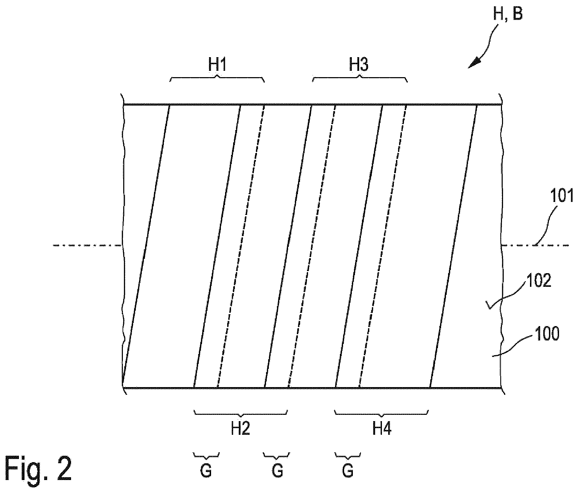

Bei der mittels der Vorrichtung 1 aufzubringenden Beschichtung (siehe Fig. 2) handelt es sich vorliegend um eine hydrophile Beschichtung B, die bei Kontakt mit Flüssigkeit einen Gleitfilm ausbildet. Der Gleitfilm unterstützt eine Beweglichkeit des Katheterschlauchs 100 bei der Katheteranlage, so dass der Katheterschlauch 100 möglichst leichtgängig an seinen Bestimmungsort in einem Führungsschlauch und/oder im Körperinneren vorgeschoben werden kann. Die Beschichtung B wird auf noch näher beschriebene Weise aus einer viskosen Beschichtungslösung S (siehe Fig. 1) auf der Mantelfläche 102 ausgebildet.

Die Vorrichtung 1 weist eine Rotationseinrichtung 10, eine Aufbringeinrichtung 20, eine Linearbewegungseinrichtung 30 und eine Steuereinrichtung 40 auf.

Bei der gezeigten Ausführungsform weist die Vorrichtung 1 zudem eine optionale Verdunstungseinrichtung 50 auf, die alternativ auch als Vernetzungseinrichtung 60 ausgebildet sein kann. Zudem weist die Vorrichtung vorliegend einen optionalen Lösungsbehälter 70 auf.

Die Rotationseinrichtung 10 ist zum Einspannen und angetriebenen Rotieren des Katheterschlauchs 100 um dessen Längsachse 101 eingerichtet. Die Rotation erfolgt mittels einer definierten Drehzahl U, d.h. Anzahl an Umdrehungen pro Zeiteinheit.

Die Aufbringeinrichtung 20 ist relativ zu der Längsachse 101 rotatorisch unbeweglich und zum Aufbringen der viskosen Beschichtungslösung S auf die Mantelfläche 102 des rotierenden Katheterschlauchs 100 eingerichtet. Das Aufbringen der viskosen Beschichtungslösung S erfolgt mit einer definierten Aufbringrate R, die ein Maß für die pro Zeiteinheit aufgebrachte Menge (Volumen und/oder Masse) der Beschichtungslösung S darstellt.

Die Linearbewegungseinrichtung 30 ist vorliegend zur angetriebenen Linearbewegung der Aufbringeinrichtung 20 entlang der Längsachse 101 eingerichtet. Die Linearbewegung erfolgt mit einer definierten Vorschubgeschwindigkeit V, d.h. Strecke entlang der Längsachse 101 pro Zeiteinheit. Bei einer in den Figuren nicht gezeigten Ausführungsform ist die Linearbewegungseinrichtung alternativ oder zusätzlich zur angetriebenen Linearbewegung der Rotationseinrichtung entlang der Längsachse eingerichtet.

Die Steuereinrichtung 40 ist zum Steuern der Drehzahl U der Rotationseinrichtung 100, der Aufbringrate R der Aufbringeinrichtung 20 und der Vorschubgeschwindigkeit V der Linearbewegungseinrichtung 30 eingerichtet. Die Steuerung der Drehzahl U und/oder der Vorschubgeschwindigkeit V kann zudem in Abhängigkeit von einem, insbesondere über der Längsachse veränderlichen, Außendurchmesser des Katheterschlauchs 100 und/oder der Mantelfläche 102 erfolgen.

Die viskose Beschichtungslösung S wird während der Vorschubbewegung der Aufbringeinrichtung 20 und unter Rotation der Rotationseinrichtung 10 und damit des Katheterschlauchs 100 auf dessen Mantelfläche 102 aufgebracht. Je nachdem, wie weit die Aufbringeinrichtung 20 entlang der Längsachse 101 vorgeschoben wird, kann die Mantelfläche 102 - mit Ausnahme eines in der Rotationseinrichtung 10 eingespannten Abschnitts - im Wesentlichen vollständig mit der viskosen Beschichtungslösung S versehen werden. Es kann aber auch nur ein Längsabschnitt der Mantelfläche 102 beschichtet werden. Zudem ist es denkbar und möglich, dass die Vorschubbewegung mehrfach wiederholt und/oder mit entgegengesetzten Richtungen durchgeführt wird. Hierdurch kann die viskose Beschichtungslösung S in mehreren Lagen aufgebracht werden.

Bei der gezeigten Ausführungsform erfolgt das Steuern der Drehzahl U, der Aufbringrate R und der Vorschubgeschwindigkeit V in Abhängigkeit der Viskosität C der aufzubringenden viskosen Beschichtungslösung S. Die Viskosität C fungiert insoweit als ein Steuerparameter für die Steuereinrichtung 40, wobei selbstverständlich auch noch weitere Steuerparameter vorgesehen sein können.

Die Viskosität C, genauer ein diese physikalische Kenngröße repräsentierender Zahlenwert, kann beispielsweise an einer in Fig. 1 nicht gezeigten Eingabeeinrichtung manuell eingegeben werden.

Alternativ oder zusätzlich kann die Viskosität C datenbasiert aus einer Speichereinrichtung abgerufen oder mittels einer Messeinrichtung gemessen werden.

Bei der gezeigten Ausführungsform ist die Steuereinrichtung 40 zu einem aufeinander abgestimmten Steuern der Drehzahl U, der Aufbringrate R und der Vorschubgeschwindigkeit V eingerichtet und zwar derart, dass die viskose Beschichtungslösung S in Form einer entlang der Längsachse 101 überlappenden Helix H auf die Mantelfläche 102 aufbringbar ist (siehe Fig. 2).

Die besagte Helix H kann auch als Wendel oder Spirale bezeichnet werden und weist in der schematischen Detaildarstellung nach Fig. 2 vier exemplarische Wicklungen oder auch Wendelungen H1, H2, H3, H4 auf. Unmittelbar zueinander benachbarte Wendelungen überlappen entlang der Längsachse 101 um ein Maß G, das auch als Überlappung bezeichnet werden kann. Durch die Überlappung kommt es zu einer vollflächigen Beschichtung.

Bei der gezeigten Ausführungsform wird die viskose Beschichtungslösung S in Bezug auf die Gravitationsrichtung von oben nach unten in Form eines hängenden Tropfens, der in den Figuren nicht im Detail gezeigt ist, an der Aufbringeinrichtung 20 ausgebracht. Der hängende Tropfen wird von dem rotierenden Katheterschlauch 100 kontaktiert, so dass dessen Mantelfläche 102 helixförmig von der viskosen Beschichtungslösung S umwickelt wird. Die Beschichtungslösung S wird folglich nicht etwa auf die Mantelfläche 102 aufgetropft oder aufgespritzt, sondern vielmehr gleichsam von der Aufbringeinrichtung "abgezogen".

Die eigentliche, im vorliegenden Fall hydrophile, Beschichtung B wird vorliegend nach einem Abtrocknen und/oder Vernetzen der auf die Mantelfläche 102 aufgebrachten viskosen Beschichtungslösung S ausgebildet.

Bei der in Fig. 1 gezeigten Ausführungsform umfasst die viskose Beschichtungslösung S eine flüchtige Lösungskomponente SF und einen in dieser gelösten Beschichtungsstoff SB. Nach Verflüchtigung der flüchtigen Lösungskomponente SF verbleibt der Beschichtungsstoff SB unter Ausbildung der Beschichtung B auf der Mantelfläche 102.

Bei der gezeigten Ausführungsform weist die Rotationseinrichtung eine Spanneinheit 11 und eine Antriebseinheit 12 auf.

Die Spanneinheit 11 ist zum endseitigen Einspannen des Katheterschlauchs 100 eingerichtet und weist zu diesem Zweck beispielsweise ein bewegliches Spannfutter, eine Klemm- oder eine Rasteinrichtung auf.

Die Antriebseinheit 12 dient der Rotation der Spanneinheit 11 und ist bevorzugt als Elektromotor ausgebildet.

Zudem kann wenigstens eine Stützeinheit vorhanden und insbesondere der Rotationseinrichtung zugeordnet sein. Die optionale Stützeinheit dient einer radialen Abstützung des zu beschichtenden Katheterschlauchs und wirkt einem Durchbiegen desselben entgegen.

Vorliegend ist die Steuereinrichtung 40 mittels einer Signalleitung 41 zum Steuern der Drehzahl U mit der Rotationseinrichtung 10, im Speziellen der Antriebseinheit 12, verbunden.

Bei der gezeigten Ausführung weist die Aufbringeinrichtung 20 eine Aufbringeinheit 21 und eine Pumpeinheit 22 auf.

Die Aufbringeinheit 21 ist vorliegend in Form einer Hohlnadel gestaltet und weist eine Austrittsöffnung (ohne Bezugszeichen) auf, durch welche die viskose Beschichtungslösung S aus der Aufbringeinrichtung 20 auf die Mantelfläche 102 austritt. Vorliegend wird der besagte (hängende) Tropfen an der Austrittsöffnung ausgebracht.

Die Pumpeinheit 22 dient einer Pumpförderung der viskosen Beschichtungslösung S durch die Aufbringeinheit 21 und deren Austrittsöffnung. Bei der gezeigten Ausführungsform ist die Pumpeinheit 22 der Aufbringeinrichtung 20 zugeordnet, was jedoch nicht bei sämtlichen Ausführungsformen der Fall ist. Die Pumpeinheit 20 ist über eine Fluidleitung (ohne Bezugszeichen) mit dem Lösungsbehälter 70 verbunden. In dem Lösungsbehälter 70 ist die aufzubringende viskose Beschichtungslösung S bevorratet. Der Lösungsbehälter 70 ist, wie eingangs bereits erwähnt, optional und nicht bei sämtlichen Ausführungsformen der Vorrichtung vorhanden. Die besagte Fluidleitung ist derart ausgebildet und angeordnet, dass eine funktionsgerechte Beweglichkeit der Aufbringeinrichtung 20 in Relation zu dem stationären Lösungsbehälter 70 gewährleistet ist.

Vorliegend ist die Steuereinrichtung 40 zum Steuern der Aufbringrate R mittels einer Signalleitung 42 mit der Aufbringeinrichtung 20 verbunden. Die Aufbringrate R ist vorliegend derart, auf die Viskosität C und die weiteren Verfahrensparameter, abgestimmt gesteuert, dass sich der bereits erwähnte (hängende) Tropfen bildet und kontinuierlich von der Aufbringeinrichtung 20 auf die Mantelfläche 102 aufgezogen wird.

Die Linearbewegungseinrichtung 30 weist bei der gezeigten Ausführungsform eine parallel zu der Längsachse 101 erstreckte Führungsachse 31 auf, die zwischen einem ersten Ende 32 und einem zweiten Ende 33 längserstreckt ist. Die Aufbringeinheit 20 ist zwischen dem ersten Ende 32 und dem zweiten Ende 33 an der Führungsachse 31 linearbeweglich geführt und angetrieben. Der Antrieb kann auf jede geeignete Weise erfolgen. Beispielsweise kann die Linearbewegungseinrichtung 30 über eine Bewegungsspindel, einen Riementrieb, eine Zahnstange oder dergleichen mit der Aufbringeinrichtung 20 zusammenwirken.

Zum Steuern der Vorschubgeschwindigkeit V ist die Steuereinrichtung 40 vorliegend über eine Signalleitung 43 mit der Linearbewegungseinrichtung 30 verbunden.

Bei der gezeigten Ausführungsform weist die Vorrichtung 1 zudem die bereits erwähnte, optionale Verdunstungseinrichtung 50 auf. Die Verdunstungseinrichtung 50 unterstützt das Abtrocknen der auf die Mantelfläche 102 aufgebrachten viskosen Beschichtungslösung S, indem der Verdunstungsprozess der flüchtigen Lösungskomponente SF in Gang gesetzt, aufrechterhalten und/oder beschleunigt wird. Die Verdunstungseinrichtung 50 kann zu diesem Zweck eine in den Figuren nicht näher gezeigte Heizeinheit, insbesondere einen Infrarot-Strahler, aufweisen, wobei alternativ oder zusätzlich auch eine Lüftereinheit vorhanden sein kann. Die Verdunstungseinrichtung 50 ist bei der gezeigten Ausführungsform im Wesentlichen über eine gesamte Länge des möglichen Vorschubwegs der Aufbringeinrichtung 20 erstreckt und auf einer der Aufbringeinrichtung 20 und der Linearbewegungseinrichtung 30 gegenüberliegenden Seite der Längsachse 101 des Katheterschlauchs 100 angeordnet. Die Anordnung ist stationär. Bei einer in den Figuren nicht gezeigten Ausführungsform kann die Verdunstungseinrichtung stattdessen vergleichsweise kürzer gemeinsam mit der Aufbringeinrichtung beweglich und um einen gewissen Längsabstand zu dieser versetzt angeordnet sein. Hierdurch wird eine im Vergleich kompaktere Bauform erreicht.

Bei der gezeigten Ausführungsform ist die Verdunstungseinrichtung 50 in die Steuerung der Vorrichtung 1 eingebunden und zu diesem Zweck über eine Signalleitung 44 mit der Steuereinrichtung 40 verbunden.

Bei der gezeigten Ausführungsform ist die Rotationseinrichtung 10 zum Einspannen und Rotieren von Invasivkomponenten mit einem Durchmesser von 0,5 mm bis 10 mm eingerichtet, im Speziellen von 0,7 mm bis 0,9 mm. Die Länge der beschichtbaren Invasivkomponenten beträgt zwischen 40 mm bis 500 mm, im Speziellen 80 mm bis 250 mm. Die mögliche Drehzahl U bewegt sich vorliegend zwischen 20 U/min bis 900 U/min.

Bei der gezeigten Ausführungsform erlaubt die Aufbringeinrichtung 20 eine Verarbeitung von viskosen Beschichtungslösungen mit einer Viskosität V von 25 cP bis 80 cP, was 25 x 10⁻³ kg/ms bis 80 x 10⁻³ kg/ms entspricht. Die möglichen Aufbringraten bewegen sich zwischen 0,5 µl/s und 10 µl/s.

Weiter sind bei der gezeigten Ausführungsform Vorschubgeschwindigkeiten V zwischen 0,5 mm/s und 50 mm/s möglich. Die Linearbewegungseinrichtung 30 ist dementsprechend eingerichtet.

Bei der im Speziellen gezeigten Beschichtung des Katheterschlauchs 100 ist eine Drehzahl U zwischen 300 U/min und 600 U/min, eine Vorschubgeschwindigkeit V zwischen 5 mm/s und 10 mm/s und eine Aufbringrate zwischen 1 µl/s und 3 µl/s vorgesehen, wobei die Viskosität V der vorliegend verwendeten viskosen Beschichtungslösung S zwischen 35 x 10⁻³ kg/ms und 55 x 10⁻³ kg/ms beträgt. Dabei liegt das Beschichtungsvolumen zwischen 1 µl/cm und 4 µl/cm.

Bei einer in den Figuren nicht gezeigten Ausführungsform beträgt die Drehzahl U 600 U/min, die Vorschubgeschwindigkeit V 10 mm/s und die Aufbringrate zwischen 0,9 µl/s und 2,5 µl/s, wobei die Viskosität V der verwendeten viskosen Beschichtungslösung S zwischen 43 x 10⁻³ kg/ms und 48 x 10⁻³ kg/ms beträgt.

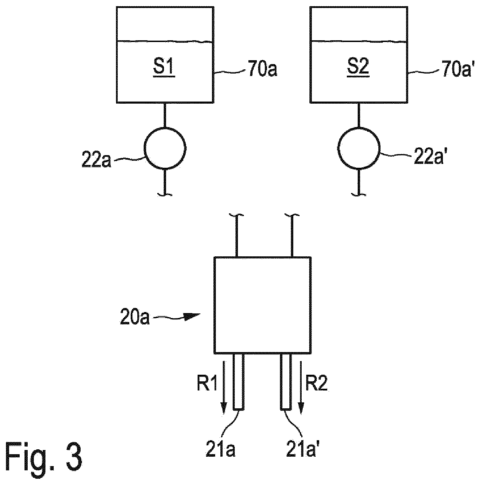

Anhand Fig. 3 ist eine unterschiedlich ausgeführte Aufbringeinrichtung 20a gezeigt, die anstelle der Aufbringeinrichtung 20 bei der Vorrichtung 1 nach Fig. 1 verwendet werden kann.

Die Aufbringeinrichtung 20a nach Fig. 3 ist zum Aufbringen einer ersten Lösungskomponente S1 und einer zweiten Lösungskomponente S2 vorgesehen, die gemeinsam die viskose Beschichtungslösung oder auch Beschichtung bilden. Solche mehrkomponentigen Beschichtungen sind insbesondere bei der hydrophilen Beschichtung von Katheterschläuchen grundsätzlich bekannt.

Die erste Lösungskomponente S1 wird in einem ersten Lösungsbehälter 70a aufbewahrt. Die zweite Lösungskomponente S2 wird in einem zweiten Lösungsbehälter 70a' aufbewahrt. Die beiden Lösungsbehälter 70a, 70a' sind über jeweils eine Fluidleitung (ohne Bezugszeichen) mit der Aufbringeinrichtung 20a verbunden. Weiter sind vorliegend separate Pumpeinheiten 22a, 22a' vorgesehen. Dabei ist jeder Fluidleitung und damit jedem der beiden Lösungsbehälter 70a, 70a' jeweils eine der beiden Pumpeinheiten 22a, 22a' zugeordnet. Die beiden Pumpeinheiten 22a, 22a' sind bei der gezeigten Variante abseits der eigentlichen Aufbringeinrichtung 20a angeordnet und relativ zu dieser stationär.

Die Aufbringeinrichtung 20a weist eine erste Aufbringeinheit 21a und eine zweite Aufbringeinheit 21a` auf. Hinsichtlich der spezifischen Gestaltung der beiden Aufbringeinheiten 21a, 21a` gilt das bereits zu Fig. 1 und der dortigen Aufbringeinheit 21 Gesagte sinngemäß. Die erste Aufbringeinheit 21a ist zum Aufbringen der ersten Lösungskomponente S1 vorgesehen und über die nicht näher bezeichnete Fluidleitung mit dem ersten Lösungsbehälter 70a verbunden. Die zweite Aufbringeinheit 21a` ist über die weitere Fluidleitung (ohne Bezugszeichen) mit dem zweiten Lösungsbehälter 70a' verbunden.

Bei der in Fig. 3 gezeigten Variante kann die erste Lösungskomponente S1 mit einer ersten Aufbringrate R1 aufgebracht werden. Die zweite Lösungskomponente S2 kann mit einer zweiten Aufbringrate R2 aufgebracht werden. Die beiden Aufbringraten R1, R2 können über eine entsprechende Steuerung der jeweiligen Pumpeinheit 22a, 22a' verändert/gesteuert werden.

Zum Ausbilden der Beschichtung werden die beiden Lösungskomponenten S1, S2 einzeln vernetzt. Zu diesem Zweck kann die Vorrichtung 1 die bereits anhand Fig. 1 erläuterte optionale und alternativ zu der Verdunstungseinrichtung 50 vorhandene Vernetzungseinrichtung 60 aufweisen. Die Vernetzungseinrichtung 60 ist zum Vernetzen und/oder Beschleunigen der Vernetzung der auf die Mantelfläche aufgebrachten Lösungskomponenten S1, S2 eingerichtet. Zu diesem Zweck kann die Vernetzungseinrichtung 60 beispielsweise eine Lichtquelle aufweisen, insbesondere eine UV-Lichtquelle.

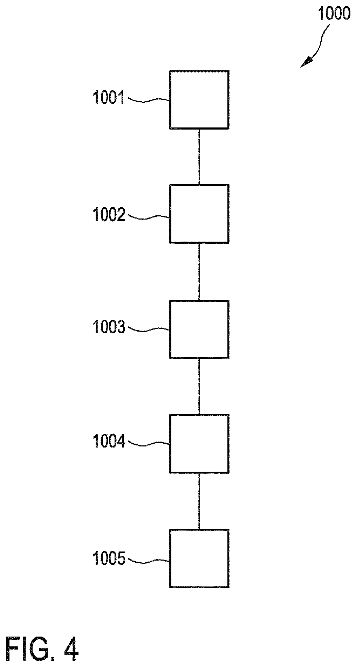

Anhand Fig. 4 ist eine Ausführungsform eines erfindungsgemäßen Verfahrens 1000 schematisch verdeutlicht. Das Verfahren 1000 kann unter Verwendung der Vorrichtung 1 nach Fig. 1 durchgeführt werden. Das Verfahren 1000 sieht ein Einspannen 1001 des Katheterschlauchs 100 in die Rotationseinrichtung 10 vor. Weiter sieht das Verfahren 1000 ein Rotieren 1002 des eingespannten Katheterschlauchs 100 mittels der Rotationseinrichtung 10 vor, wobei die Rotation mit der Drehzahl U erfolgt. Weiter sieht das Verfahren 1000 ein Aufbringen 1003 der viskosen Beschichtungslösung S auf die Mantelfläche 102 des rotierenden Katheterschlauchs 100 vor. Die viskose Beschichtungslösung S wird hierbei mittels der Aufbringeinrichtung 20 aufgebracht. Dies mit der Aufbringrate R. Weiter sieht das Verfahren 1000 ein lineares Bewegen 1004 der Aufbringeinrichtung 20 entlang der Längsachse 101 des rotierenden Katheterschlauchs 100 vor. Die Aufbringeinrichtung 20 wird hierbei mittels der Linearbewegungseinrichtung 30 mit einer definierten Vorschubgeschwindigkeit V linear entlang der Längsachse 101 bewegt. Weiter sieht das Verfahren 1000 ein Steuern 1005 der Drehzahl U, der Aufbringrate R und der Vorschubgeschwindigkeit V mittels der Steuereinrichtung 40 vor. Dabei werden die Drehzahl U, die Aufbringrate R und die Vorschubgeschwindigkeit V in Abhängigkeit der Viskosität C der viskosen Beschichtungslösung S derart aufeinander abgestimmt gesteuert, dass die viskose Beschichtungslösung S vollflächig in Form der besagten, entlang der Längsachse 101 überlappenden, Helix H auf die Mantelfläche 102 aufgebracht wird.

## Patentansprüche

1. Vorrichtung (1) zum Beschichten einer medizinischen Invasivkomponente (100) mit einer Längsachse (101) und einer um die Längsachse (101) rotationssymmetrischen sowie gerade längserstreckten Mantelfläche (102), aufweisend
eine Rotationseinrichtung (10), die eingerichtet ist zum Einspannen und angetriebenen Rotieren der Invasivkomponente (100) um deren Längsachse (101) mit einer definierten Drehzahl (U),
eine Aufbringeinrichtung (20, 20a), die relativ zu der Längsachse (101) rotatorisch unbeweglich ist, und die eingerichtet ist zum Aufbringen einer viskosen Beschichtungslösung (S) auf die Mantelfläche (102) der rotierenden Invasivkomponente (100) mit einer definierten Aufbringrate (R),
eine Linearbewegungseinrichtung (30), die eingerichtet ist zur angetriebenen relativen Linearbewegung zwischen der Aufbringeinrichtung (20, 20a) und der Rotationseinrichtung (10) mit einer definierten Vorschubgeschwindigkeit (V) entlang der Längsachse (101) der rotierenden Invasivkomponente (100),
eine Steuereinrichtung (40), die eingerichtet ist zum Steuern der Drehzahl (U) der Rotationseinrichtung (10), der Aufbringrate (R) der Aufbringeinrichtung (20, 20a) und der Vorschubgeschwindigkeit (V) der Linearbewegungseinrichtung (30),
**dadurch gekennzeichnet, dass** die Steuereinrichtung (40) derart zu einem aufeinander abgestimmten Steuern der Drehzahl (U), der Aufbringrate (R) und der Vorschubgeschwindigkeit (V) in Abhängigkeit der Viskosität (C) der viskosen Beschichtungslösung (S) eingerichtet ist, dass die viskose Beschichtungslösung (S) vollflächig in Form einer entlang der Längsachse (101) überlappenden Helix (H) auf die Mantelfläche (102) aufbringbar ist.

2. Vorrichtung (1) nach Anspruch 1, **dadurch gekennzeichnet, dass** die aufzubringende viskose Beschichtungslösung (S) aus mehreren separat bevorrateten Lösungskomponenten (S1, S2) gebildet ist, wobei die Aufbringeinrichtung (20a) zum gleichzeitigen Aufbringen der mehreren Lösungskomponenten (S1, S2) eingerichtet ist und für jede der mehreren Lösungskomponenten (S1, S2) jeweils eine Aufbringeinheit (21a, 21a') aufweist.

3. Vorrichtung (1) nach Anspruch 1 oder 2, **dadurch gekennzeichnet, dass** eine Verdunstungseinrichtung (50) vorhanden und zum Verdunsten und/oder Beschleunigen einer Verdunstung einer flüchtigen Lösungskomponente (SF) der auf die Mantelfläche (102) aufgebrachten viskosen Beschichtungslösung (S) eingerichtet ist.

4. Vorrichtung (1) nach einem der vorhergehenden Ansprüche, **dadurch gekennzeichnet, dass** eine Vernetzungseinrichtung (60) vorhanden und zum Vernetzen und/oder Beschleunigen einer Vernetzung von zu vernetzenden Lösungskomponenten (S1, S2) der auf die Mantelfläche (102) aufgebrachten viskosen Beschichtungslösung (S) eingerichtet ist.

5. Vorrichtung (1) nach einem der vorhergehenden Ansprüche, **dadurch gekennzeichnet, dass** die Rotationseinrichtung (10) eingerichtet ist zum Einspannen und angetriebenen Rotieren von Invasivkomponenten mit einem Durchmesser von 0,5 mm bis 10 mm und einer Länge von 40 mm bis 500 mm und mit einer Drehzahl von 20 U/min bis 900 U/min, bevorzugt mit einer Drehzahl von 300 U/min bis 600 U/min.

6. Vorrichtung (1) nach einem der vorhergehenden Ansprüche, **dadurch gekennzeichnet, dass** die Aufbringeinrichtung (20, 20a) eingerichtet ist zum Aufbringen viskoser Beschichtungslösungen mit einer Viskosität (C) von 25 x 10⁻³ kg/ms bis 80 x 10⁻³ kg/ms, bevorzugt von 35 x 10⁻³ kg/ms bis 55 x 10⁻³ kg/ms, mit einer Aufbringrate von 0,5 µl/s bis 10 µl/s, bevorzugt von 1 µl/s bis 3 µl/s.

7. Vorrichtung (1) nach einem der vorhergehenden Ansprüche, **dadurch gekennzeichnet, dass** die Linearbewegungseinrichtung (30) eingerichtet ist zur angetriebenen Linearbewegung der Aufbringeinrichtung (20, 20a) und/oder der Rotationseinrichtung (10) mit einer Vorschubgeschwindigkeit (V) zwischen 0,5 mm/s und 50 mm/s, bevorzugt zwischen 5 mm/s und 10 mm/s.

8. Verfahren (1000) zum Beschichten einer medizinischen Invasivkomponente (100) mit einer Längsachse (101) und einer um die Längsachse (101) rotationssymmetrischen sowie gerade längserstreckten Mantelfläche (102), aufweisend die Schritte:
Einspannen (1001) der Invasivkomponente (100) in eine Rotationseinrichtung (10);
Rotieren (1002) der eingespannten Invasivkomponente (100) mittels der Rotationseinrichtung (10), wobei die Rotation mit einer definierten Drehzahl (U) erfolgt;
Aufbringen (1003) einer viskosen Beschichtungslösung (S) auf die Mantelfläche (102) der rotierenden Invasivkomponente (100), wobei die viskose Beschichtungslösung (S) mittels einer Aufbringeinrichtung (20, 20a), die relativ zu der Längsachse (101) rotatorisch unbeweglich ist, und mit einer definierten Aufbringrate (R) aufgebracht wird;
lineares Bewegen (1004) der Aufbringeinrichtung (20, 20a) und/oder der Rotationseinrichtung (10) entlang der Längsachse (101) der rotierenden Invasivkomponente (100), wobei die Aufbringeinrichtung (20, 20a) mittels einer Linearbewegungseinrichtung (30) und mit einer definierten Vorschubgeschwindigkeit (V) relativ zu der Rotationseinrichtung (10) linear bewegt wird und/oder umgekehrt;
Steuern (1005) der Drehzahl (U), der Aufbringrate (R) und der Vorschubgeschwindigkeit (V) mittels einer Steuereinrichtung (40),
**dadurch gekennzeichnet, dass** die Drehzahl (U), die Aufbringrate (R) und die Vorschubgeschwindigkeit (V) in Abhängigkeit der Viskosität (C) der viskosen Beschichtungslösung (S) derart aufeinander abgestimmt gesteuert werden, dass die viskose Beschichtungslösung (S) vollflächig in Form einer entlang der Längsachse (101) überlappenden Helix (H) auf die Mantelfläche (102) aufgebracht wird.

9. Verfahren (1000) nach Anspruch 8, **dadurch gekennzeichnet, dass** die Drehzahl (U) zwischen 20 U/min und 900 U/min, bevorzugt zwischen 300 U/min bis 600 U/min, beträgt.

10. Verfahren (1000) nach Anspruch 8 oder 9, **dadurch gekennzeichnet, dass** die Viskosität (C) zwischen 25 x 10⁻³ kg/ms und 80 x 10⁻³ kg/ms, bevorzugt zwischen 35 x 10⁻³ kg/ms und 55 x 10⁻³ kg/ms, beträgt.

11. Verfahren (1000) nach einem der Ansprüche 8 bis 10, **dadurch gekennzeichnet, dass** die Aufbringrate (R) zwischen 0,5 µl/s und 10 µl/s, bevorzugt zwischen 1 µl/s und 3 µl/s, beträgt.

12. Verfahren (1000) nach einem der Ansprüche 8 bis 11, **dadurch gekennzeichnet, dass** die Vorschubgeschwindigkeit (V) zwischen 0,5 mm/s und 50 mm/s, bevorzugt zwischen 5 mm/s und 10 mm/s, beträgt.

13. Verfahren (1000) nach einem der Ansprüche 8 bis 12, **dadurch gekennzeichnet, dass** die Drehzahl (U) zwischen 300 U/min und 600 U/min beträgt, dass die Viskosität (C) zwischen 35 x 10⁻³ kg/ms und 55 x 10⁻³ kg/ms beträgt, dass die Aufbringrate (R) zwischen 1 µl/s und 3 µl/s beträgt, und dass Vorschubgeschwindigkeit (V) zwischen 5 mm/s und 10 mm/s beträgt.

14. Verfahren (1000) nach einem der Ansprüche 8 bis 13, **dadurch gekennzeichnet, dass** die viskose Beschichtungslösung (S) in Bezug auf die Gravitationsrichtung von oben nach unten in Form eines hängenden Tropfens an der Aufbringeinrichtung (20, 20a) ausgebracht wird, wobei der hängende Tropfen von der rotierenden Invasivkomponente (100) kontaktiert wird, wodurch deren Mantelfläche (102) von der viskosen Beschichtungslösung (S) helixförmig umwickelt wird.

15. Verfahren (1000) nach einem der Ansprüche 8 bis 13, **dadurch gekennzeichnet, dass** die viskose Beschichtungslösung (S) in Bezug auf die Gravitationsrichtung von unten nach oben in Form eines stehenden Tropfens an der Aufbringeinrichtung (20, 20a) ausgebracht wird, wobei der stehende Tropfen von der rotierenden Invasivkomponente (100) kontaktiert wird, wodurch deren Mantelfläche (102) von der viskosen Beschichtungslösung (S) helixförmig umwickelt wird.

## Claims

1. Apparatus (1) for coating a medical invasive component (100) having a longitudinal axis (101) and a lateral face (102) which is rotationally symmetrical about the longitudinal axis (101) and extends longitudinally in a straight manner, the apparatus comprising
a rotation device (10) which is configured to clamp the invasive component (100) and rotate the latter in a driven manner about its longitudinal axis (101) at a defined rotational speed (U),
an application device (20, 20a) which is rotationally immovable relative to the longitudinal axis (101) and which is configured to apply a viscous coating solution (S) onto the lateral face (102) of the rotating invasive component (100) at a defined application rate (R),
a linear movement device (30) which is configured for driven relative linear movement between the application device (20, 20a) and the rotation device (10) at a defined speed of advance (V) along the longitudinal axis (101) of the rotating invasive component (100),
a control device (40) which is configured to control the rotational speed (U) of the rotation device (10), the application rate (R) of the application device (20, 20a) and the speed of advance (V) of the linear movement device (30),
**characterized in that** the control device (40) is configured to control the rotational speed (U), the application rate (R) and the speed of advance (V) in a coordinated manner depending on the viscosity (C) of the viscous coating solution (S), such that the viscous coating solution (S) can be applied over the full surface of the lateral face (102) in the form of a helix (H) which overlaps along the longitudinal axis (101).

2. Apparatus (1) according to Claim 1, **characterized in that** the viscous coating solution (S) to be applied is formed from a plurality of separately stored solution components (S1, S2), wherein the application device (20a) is configured for simultaneous application of the plurality of solution components (S1, S2) and has an application unit (21a, 21a') for each of the plurality of solution components (S1, S2).

3. Apparatus (1) according to Claim 1 or 2, **characterized in that** an evaporation device (50) is present and is configured for evaporating and/or accelerating evaporation of a volatile solution component (SF) of the viscous coating solution (S) applied to the lateral face (102).

4. Apparatus (1) according to any one of the preceding claims, **characterized in that** a crosslinking device (60) is present and is configured for crosslinking and/or accelerating crosslinking of crosslinkable solution components (S1, S2) of the viscous coating solution (S) applied to the lateral face (102).

5. Apparatus (1) according to any one of the preceding claims, **characterized in that** the rotation device (10) is configured for the clamping and driven rotation of invasive components having a diameter of 0.5 mm to 10 mm and a length of 40 mm to 500 mm and at a rotational speed of 20 rpm to 900 rpm, preferably at a rotational speed of 300 rpm to 600 rpm.

6. Apparatus (1) according to any one of the preceding claims, **characterized in that** the application device (20, 20a) is configured for applying viscous coating solutions having a viscosity (C) of 25 x 10⁻³ kg/ms to 80 x 10⁻³ kg/ms, preferably 35 x 10⁻³ kg/ms to 55 x 10⁻³ kg/ms, at an application rate of 0.5 µl/s to 10 µl/s, preferably 1 µl/s to 3 µl/s.

7. Apparatus (1) according to any one of the preceding claims, **characterized in that** the linear movement device (30) is configured for the driven linear movement of the application device (20, 20a) and/or of the rotation device (10) at a speed of advance (V) of between 0.5 mm/s and 50 mm/s, preferably of between 5 mm/s and 10 mm/s.

8. Method (1000) for coating a medical invasive component (100) having a longitudinal axis (101) and a lateral face (102) which is rotationally symmetrical about the longitudinal axis (101) and extends longitudinally in a straight manner, the method comprising the following steps:
clamping (1001) the invasive component (100) into a rotation device (10);
rotating (1002) the clamped invasive component (100) by means of the rotation device (10), the rotation taking place at a defined rotational speed (U);
applying (1003) a viscous coating solution (S) to the lateral face (102) of the rotating invasive component (100), wherein the viscous coating solution (S) is applied by means of an application device (20, 20a), which is rotationally immovable relative to the longitudinal axis (101), and at a defined application rate (R);
linearly moving (1004) the application device (20, 20a) and/or the rotation device (10) along the longitudinal axis (101) of the rotating invasive component (100), wherein the application device (20, 20a) is moved linearly by means of a linear movement device (30) and at a defined speed of advance (V) relative to the rotation device (10), and/or vice versa;
controlling (1005) the rotational speed (U), the application rate (R) and the speed of advance (V) by means of a control device (40),
**characterized in that** the rotational speed (U), the application rate (R) and the speed of advance (V) are controlled in a coordinated manner depending on the viscosity (C) of the viscous coating solution (S), such that the viscous coating solution (S) is applied over the full surface of the lateral face (102) in the form of a helix (H) which overlaps along the longitudinal axis (101).

9. Method (1000) according to Claim 8, **characterized in that** the rotational speed (U) is between 20 rpm and 900 rpm, preferably between 300 rpm and 600 rpm.

10. Method (1000) according to Claim 8 or 9, **characterized in that** the viscosity (C) is between 25 x 10⁻³ kg/ms and 80 x 10⁻³ kg/ms, preferably between 35 x 10⁻³ kg/ms and 55 x 10⁻³ kg/ms.

11. Method (1000) according to any one of Claims 8 to 10, **characterized in that** the application rate (R) is between 0.5 µl/s and 10 µl/s, preferably between 1 µl/s and 3 µl/s.

12. Method (1000) according to any one of Claims 8 to 11, **characterized in that** the speed of advance (V) is between 0.5 mm/s and 50 mm/s, preferably between 5 mm/s and 10 mm/s.

13. Method (1000) according to any one of Claims 8 to 12, **characterized in that** the rotational speed (U) is between 300 rpm and 600 rpm, **in that** the viscosity (C) is between 35 x 10⁻³ kg/ms and 55 x 10⁻³ kg/ms, **in that** the application rate (R) is between 1 µl/s and 3 µl/s, and **in that** the speed of advance (V) is between 5 mm/s and 10 mm/s.

14. Method (1000) according to any one of Claims 8 to 13, **characterized in that**, in relation to the gravitational direction, the viscous coating solution (S) is discharged downward in the form of a hanging drop on the application device (20, 20a), wherein the hanging drop is contacted by the rotating invasive component (100), as a result of which the viscous coating solution (S) winds helically around the lateral face (102).

15. Method (1000) as claimed in any one of Claims 8 to 13, **characterized in that**, in relation to the gravitational direction, the viscous coating solution (S) is discharged upward in the form of a standing drop on the application device (20, 20a), wherein the standing drop is contacted by the rotating invasive component (100), as a result of which the viscous coating solution (S) winds helically around the lateral face (102).

## Revendications

1. Dispositif (1) de revêtement d'un composant invasif (100) médical avec un axe longitudinal (101) et une surface extérieure (102) symétrique en rotation autour de l'axe longitudinal (101) et s'étendant longitudinalement en ligne droite, comportant
un système de rotation (10), qui est mis au point pour enserrer et faire tourner de manière entraînée le composant invasif (100) autour de son axe longitudinal (101) à une vitesse de rotation (U) définie,
un système d'application (20, 20a), qui est immobile en rotation par rapport à l'axe longitudinal (101) et qui est mis au point pour appliquer une solution de revêtement (S) visqueuse sur la surface extérieure (102) du composant invasif (100) en rotation à un taux d'application (R) défini,
un système de déplacement linéaire (30), qui est mis au point pour se déplacer linéairement de manière entraînée entre le système d'application (20, 20a) et le système de rotation (10) à une vitesse d'avancement (V) définie le long de l'axe longitudinal (101) du composant invasif (100) en rotation,
un système de commande (40), qui est mis au point pour commander la vitesse de rotation (U) du système de rotation (10), le taux d'application (R) du système d'application (20, 20a) et la vitesse d'avancement (V) du système de déplacement linéaire (30),
**caractérisé en ce que** le système de commande (40) est mis au point pour commander de manière adaptée les uns aux autres la vitesse de rotation (U), le taux d'application (R) et la vitesse d'avancement (V) en fonction de la viscosité (C) de la solution de revêtement (S) visqueuse de telle manière que la solution de revêtement (S) visqueuse peut être appliquée sur la surface extérieure (102) sur la totalité de la surface sous la forme d'une hélice (H) de chevauchement le long de l'axe longitudinal (101).

2. Dispositif (1) selon la revendication 1, **caractérisé en ce que** la solution de revêtement (S) visqueuse à appliquer est obtenue à partir de plusieurs composants de solution (S1, S2) stockés séparément, le système d'application (20a) étant mis au point pour appliquer simultanément les plusieurs composants de solution (S1, S2) et comportant pour chacun des plusieurs composants de solution (S1, S2) respectivement une unité d'application (21a, 21a').

3. Dispositif (1) selon la revendication 1 ou 2, **caractérisé en ce qu'**un système d'évaporation (50) est présent pour évaporer et/ou accélérer une évaporation d'un composant de solution volatile (SF) de la solution de revêtement (S) visqueuse appliquée sur la surface extérieure (102).

4. Dispositif (1) selon l'une des revendications précédentes, **caractérisé en ce qu'**un système de réticulation (60) est présent pour réticuler et/ou accélérer une réticulation de composants de solution (S1, S2) à réticuler de la solution de revêtement (S) visqueuse appliquée sur la surface extérieure (102).

5. Dispositif (1) selon l'une des revendications précédentes, **caractérisé en ce que** le système de rotation (10) est mis au point pour enserrer et faire tourner de manière entraînée des composants invasifs avec un diamètre de 0,5 mm à 10 mm et une longueur de 40 mm à 500 mm et à une vitesse de rotation de 20 t/min à 900 t/min, de manière préférée à une vitesse de rotation de 300 t/min à 600 t/min.

6. Dispositif (1) selon l'une des revendications précédentes, **caractérisé en ce que** le système d'application (20, 20a) est mis au point pour appliquer des solutions de revêtement visqueuses avec une viscosité (C) de 25 x 10⁻³ kg/ms à 80 x 10⁻³ kg/ms, de manière préférée de 35 x 10⁻³ kg/ms à 55 x 10⁻³ kg/ms, à un taux d'application de 0,5 µl/s à 10 µl/s, de manière préférée de 1 µl/s à 3 µl/s.

7. Dispositif (1) selon l'une des revendications précédentes, **caractérisé en ce que** le système de déplacement linéaire (30) est mis au point pour déplacer linéairement de manière entraînée le système d'application (20, 20a) et/ou le système de rotation (10) à une vitesse d'avancement (V) comprise entre 0,5 mm/s et 50 mm/s, de manière préférée entre 5 mm/s et 10 mm/s.

8. Procédé (1000) de revêtement d'un composant invasif (100) médical avec un axe longitudinal (101) et une surface extérieure (102) symétrique en rotation autour de l'axe longitudinal (101) et s'étendant longitudinalement en ligne droite, présentant les étapes :
enserrage (1001) du composant invasif (100) dans un système de rotation (10) ;
rotation (1002) du composant invasif (100) enserré au moyen du système de rotation (10), la rotation étant effectuée à une vitesse de rotation (U) définie ;
application (1003) d'une solution de revêtement (S) visqueuse sur la surface extérieure (102) du composant invasif (100) en rotation, la solution de revêtement (S) visqueuse étant appliquée au moyen d'un système d'application (20, 20a), qui est immobile en rotation par rapport à l'axe longitudinal (101), et à un taux d'application (R) défini ;
déplacement linéaire (1004) du système d'application (20, 20a) et/ou du système de rotation (10) le long de l'axe longitudinal (101) du composant invasif (100) en rotation, le système d'application (20, 20a) étant déplacé linéairement au moyen d'un système de déplacement linéaire (30) et à une vitesse d'avancement (V) définie par rapport au système de rotation (10) et/ou inversement ;
commande (1005) de la vitesse de rotation (U), du taux d'application (R) et de la vitesse d'avancement (V) au moyen d'un système de commande (40),
**caractérisé en ce que** la vitesse de rotation (U), le taux d'application (R) et la vitesse d'avancement (V) sont commandés de manière adaptée les uns sur les autres en fonction de la viscosité (C) de la solution de revêtement (S) visqueuse de telle manière que la solution de revêtement (S) visqueuse est appliquée sur la surface extérieure (102) sur la totalité de la surface sous la forme d'une hélice (H) de chevauchement le long de l'axe longitudinal (101).

9. Procédé (1000) selon la revendication 8, **caractérisé en ce que** la vitesse de rotation (U) est comprise entre 20 t/min et 900 t/min, de manière préférée entre 300 t/min à 600 t/min,

10. Procédé (1000) selon la revendication 8 ou 9, **caractérisé en ce que** la viscosité (C) est comprise entre 25 x 10⁻³ kg/ms et 80 x 10⁻³ kg/ms, de manière préférée entre 35 x 10⁻³ kg/ms et 55 x 10⁻³ kg/ms.

11. Procédé (1000) selon l'une des revendications 8 à 10, **caractérisé en ce que** le taux d'application (R) est compris entre 0,5 µl/s et 10 µl/s, de manière préférée entre 1 µl/s et 3 µl/s.

12. Procédé (1000) selon l'une des revendications 8 à 11, **caractérisé en ce que** la vitesse d'avancement (V) est comprise entre 0,5 mm/s et 50 mm/s, de manière préférée entre 5 mm/s et 10 mm/s.

13. Procédé (1000) selon l'une des revendications 8 à 12, **caractérisé en ce que** la vitesse de rotation (U) est comprise entre 300 t/min et 600 t/min, que la viscosité (C) est comprise entre 35 x 10-3 kg/ms et 55 x 10-3 kg/ms, que le taux d'application (R) est compris entre 1 µl/s et 3 µl/, et que la vitesse d'avancement (V) est comprise entre 5 mm/s et 10 mm/s.

14. Procédé (1000) selon l'une des revendications 8 à 13, **caractérisé en ce que** la solution de revêtement (S) visqueuse est appliquée, par rapport au sens de gravitation, du haut vers le bas sous la forme d'une gouttelette suspendue sur le système d'application (20, 20a), la gouttelette suspendue étant mise en contact avec le composant invasif (100) en rotation ce qui permet d'enrober la surface extérieure (102) de celui-ci sous la forme d'une hélice de la solution de revêtement (S) visqueuse.

15. Procédé (1000) selon l'une des revendications 8 à 13, **caractérisé en ce que** la solution de revêtement (S) visqueuse est appliquée, par rapport au sens de gravitation, de bas vers en haut sous la forme d'une gouttelette verticale sur le système d'application (20, 20a), la gouttelette verticale étant mise en contact avec le composant invasif (100) en rotation ce qui permet d'enrober la surface extérieure (102) de celui-ci sous la forme d'une hélice de la solution de revêtement (S) visqueuse.
